Europäisches Patentamt

(19) European Patent Office

Office européen des brevets

(11) **EP 0 548 807 B1**

(12) **EUROPEAN PATENT SPECIFICATION**

(45) Date of publication and mention
of the grant of the patent:
**24.04.1996 Bulletin 1996/17**

(51) Int. Cl.$^6$: **C07C 37/60**, C07C 39/08,
C07C 41/26, C07C 43/23

(21) Application number: **92121494.6**

(22) Date of filing: **17.12.1992**

(54) **Method for preparing dihydric phenols**

Verfahren zur Herstellung von dihydrischen Phenolen

Procédé pour la préparation de phénols dihydriques

(84) Designated Contracting States:
**DE FR GB IT**

(30) Priority: **20.12.1991 JP 338304/91**

(43) Date of publication of application:
**30.06.1993 Bulletin 1993/26**

(73) Proprietor: **MITSUBISHI GAS CHEMICAL
COMPANY, INC.
Chiyoda-ku, Tokyo (JP)**

(72) Inventors:
• **Sugai, Ryuji
Niigata City (JP)**
• **Kondo, Osamu,
Mitsubishi Gas
Tsukuba City (JP)**
• **Konishi, Akihiko
Kashiwa City (JP)**

(74) Representative: **Goddar, Heinz J., Dr. et al
FORRESTER & BOEHMERT
Franz-Joseph-Strasse 38
D-80801 München (DE)**

(56) References cited:
**EP-A- 0 314 582        FR-A- 2 657 346**

• **CHEMICAL ABSTRACTS, vol. 112, no. 24, 11 June
1990, Columbus, Ohio, US; abstract no. 219197j,
J.S. REDDY ET AL. 'Titanium silicatite-2:
synthesis,characterization and catalytic
properties.' page 128 ;column 1 ;**

**Description**

BACKGROUND OF THE INVENTION

1. Field of the Invention

The present invention relates to a novel method for preparing dihydric phenols, such as hydroquinone and catechol, useful as a raw material for several industrial chemicals, pharmaceuticals, agricultural chemicals and perfumes, etc.

2. Description of the Related Art

Methods for preparing dihydric phenols, such as a hydroquinone and catechol, which are produced by oxidizing phenols with hydrogen peroxide in the presence of many catalyst systems have been studied for a long time.

For example, the following references describe the oxidation of phenols in the presence of various catalyst:

Japanese Patent Publication Kokai No. 50-130727 describes phenol oxidation in the presence of ketones, such as, 4-methyl-2-pentanone and methyl phenyl ketone, etc., and sulfates, such as an aluminium sulfate;

Japanese Patent Publication Kokoku No. 56-47891 describes phenol oxidation in the presence of strong acid mineral acids, such as a perchloric acid;

U.S. Patent No. 3,914,323 describes a method known as the Fenton Method in which phenols are oxidized in the presence of iron or cobalt salts;

U.K. Patent No. 2,116,974 and EP Patent No. 314,582 describe a method in which phenols are oxidized in the presence of synthetic zeolites containing Ti atoms as a catalyst;

EP Patent No. 299,893 describes a method in which phenols are oxidized in the presence of a lamellar acid clay as a catalyst; and

EP Patent No. 132,783 describes a method in which phenols are oxidized in the presence of a strong acid type ion-exchange resin as a catalyst, etc.

All of the above methods produce a high yield of dihydric phenols _versus_ hydrogen peroxide, hut have a very serious problem, i.e., for example, a hydroquinone and a catechol are co-produced when phenols are oxidized with hydrogen peroxide. That is, the para- to ortho-isomer ratio (P/O) of dihydric phenols in the above cases of the use of a ketoneperoxide catalysts system, mineral acid system and the Fenton reaction are 0.4-0.7; therefore, ortho-isomers, which are in little demand, are produced in large amounts compared with the amounts of para-isomers. For this reason, there is the drawback that the production amounts and the production costs of the para-isomers are very adversely influenced by the market for ortho-isomers, e.g., the market for catechols. EP Patent No. 314,582 discloses that an improvement of the para-selectivity can be accomplished by using a titanosilicate catalyst having the same crystalline structure as ZSM-5 zeolite; in this case, however, the P/O ratio is also around 1. In the case where lamellar clay is used (EP Patent No. 299,893), an example is set forth in which the P/O ratio was improved to 1.86; but the conversion ratio of hydrogen peroxide was small, and the method, therefore, is not practical. Further, there is a report that the P/O ratio can be improved to 12.5 when a strong acid type ion-exchange resin containing a cation of transition metal, such as Ti or V etc., is used as a catalyst; the yield of dihydric phenols _versus_ hydrogen peroxide was small, however, and the result, therefore, is unsatisfactory. A method of obtaining para-isomers, utilizing the shape selectivity of ZSM-5 zeolite (U.S. Patent No. 4,578,521) has recently been disclosed. Although the present inventors tried to hydroxylate a phenol according to the method described in the above U.S. Patent, satisfactory yield and selectivity could not be obtained, and the method was not sufficiently reproducible.

SUMMARY OF THE INVENTION

The inventors of the present invention have extensively studied with the objectives of producing dihydric phenols, the two hydroxyl groups of which have a para-relationship to each other, in high yields and high selectivity. They have found that the above objects of the present invention can be easily achieved by combining cyclic ethers in the reaction systems in which the hydroxylation reaction of phenols, in the following understood as meaning phenols or anisoles, by a hydrogen peroxide is carried out using a crystalline titanosilicate as a catalyst, and have thus accomplished the present invention.

The novel preparation methods of dihydric phenols according to the present invention are as follows:

a) the phenols are reacted with hydrogen peroxide in the presence of a crystalline titanosilicate and cyclic ethers.

b) preferably, said titanosilicate is a pentasyl type titanosilicate.

c) as a further preference, the cyclic ethers are ones selected from the grouß consisting of 1,3-dioxolane, tetrahydrofuran, tetrahydropyran, 1,3-dioxane, 1,4-dioxane and 1,3,5-trioxane.

d) the most preferred cyclic ether is 1,4-dioxane.

e) said cyclic ethers are preferably used in an amount of 0.04-1.2 moles per 1 mole of phenols.

f) more preferably, the cyclic ethers are used in an amount of 0.1-0.8 moles per 1 mole of phenols.

g) the reaction is preferably carried out in a polar solvent which contains cyclic ethers.

h) the P/O ratio is preferably controlled by varying the ratio of the amount of cyclic ether to the amount of the phenols.

i) the polar solvent is selected from the group consisting of water, acetone, methanol, acetonitrile and ethanol. The preferred polar solvent is water.

j) the hydrogen peroxide is used in an amount of not more than 0.5, and preferably is used in an amount of not more than 0.3 moles per 1 mole of the phenols.

k) the reaction temperature is in the range of from 50° to 150°C.

l) the titanosilicate catalyst is used in an amount of from 0.02 to 1.8 g and preferably is used in an amount of from 0.04 to 0.5 g per 1 g of the phenols; and

m) the phenols used in the reaction are selected from the group consisting of phenol and anisole.

DESCRIPTION OF THE PREFERRED EMBODIMENTS

The present invention will be described below in more detail.

In the present invention, crystalline titanosilicates, the general formula of which is shown by $xTiO_2 \cdot (1-x)SiO_2$ ($0.0001 < x < 0.5$, more preferably $0.001 < x < 0.05$), are used as catalysts. Preferably, the crystalline titanosilicate used will have a crystalline structure similar to ZSM-5; such materials are called generically "pentasyl type"; their general formulae are shown by $xTiO_2 \cdot (1-x)SiO_2$ (wherein, $0.0001 < x < 0.5$, more preferably $0.001 < x < 0.05$) are particularly preferably used.

Known methods for the preparation of the catalyst include either the method in which a reaction mixture containing a silicon source, a titanium source, nitrogen containing compounds and water is prepared; then hydrothermal synthesis is carried out, or the method in which a zeolite, such as, ZSM-5, etc., is de-alumininated in the presence of chloric acid, and Ti atoms are incorporated (B. Kraushaar and J.H.C. Van Hooff, Catl. Lett., 1(4), 81, (1988)), etc. In the present invention, although either method may be used, the former method is described in detail hereinafter.

As a silicon source, a tetraalkyl orthosilicate or a colloidal silica, may be used. As a tetraalkyl orthosilicate, a tetraethyl orthosilicate is preferably used. As a titanium source, a tetraalkyl orthotitanate, or a hydrolytic halogenated titanium compound, such as a $TiOCl_2$ may be used. As a tetraalkyl orthotitanate, a tetraethyl orthotitanate, or a tetrabutyl orthotitanate may be preferably used. As a nitrogen containing compound, a tetraalkylammonium ion, preferably a tetrapropylammonium hydroxide, or a tetrabutylammonium hydroxide may be used. Further, as a nitrogen containing compound, there may be used a titanosilicate having various crystalline forms which may be synthesized by using amines which are generally used in the preparation of a zeolite catalyst, such as a diethanolamine or a piperidine.

In preparing the titanosilicate of the present invention, the molar ratio in the charge is preferably Si/Ti=10-50, $H_2O$/Si=10-100, and the nitrogen containing compound/Si=0.05-1. The crystalline titanosilicate catalyst is obtained from the reaction products obtained by mixing the above charge and subjecting the charge to hydrothermal synthesis in an autoclave at 100-220°C for 1-1000 hours; the thus obtained solid is washed with ion-exchanged water, dried, and calcined in air at 400-600°C for 1-10 hours.

In the case when a tetrapropylammonium hydroxide is used as the nitrogen-containing compound, a titanosilicate having a silicalite-1 type structure (abbreviated as TS-1) is obtained (Japanese Patent Publication Kokoku No. 1-42889), and in case when a tetrabutylammonium hydroxide is used, a titanosilicate having a silicalite-2 type structure (abbreviated as TS-2) is obtained (J.S. Reddy et al., Appl. Catal., 5 8(2), L1-L4,(1990)).

The amount of the titanosilicate catalyst used is in the range of 0.02-0.8 g, preferably 0.04-0.5 g, per 1 g of phenols. In the case of the use of the smaller amount, the reaction rate is slower; in the case of the larger amount, it is not economical. So, the above range is the most practical range to use.

As the phenol used in the present invention, a phenol, an anisole, a cresol and a xylenol are preferably used; phenol is especially preferred.

The concentration of hydrogen peroxide is not critical; however, a 30-60 weight % aqueous solution, which is readily, commercially available, is preferably used.

The amount of hydrogen peroxide is preferably not more than 0.5 moles, and more preferably, is not more than 0.3 moles per 1 mole of the phenol, in order to avoid contributing to the side reaction. The whole quantity of hydrogen peroxide may be added at once, but, it is preferable to add it intermittently or continuously in order to inhibit the formation of excess heat and the undesired side reaction.

As described in the above, the characteristic of the preparation method of the present invention is that cyclic ethers are also present when the phenol is hydroxylated in the presence of a crystalline titanosilicate as a catalyst. As the cyclic ether, a 1,3-dioxane, a 1,4-dioxane, a 1,3,5-trioxane, are exemplified. Among those, the 1,4-dioxane is especially preferred.

The amount of the cyclic ether used is in the range of 0.04-1.2 moles per 1 mole of phenol, and preferably, in the range from 0.1 to 0.8. In case an amount less than the stated range is used a sufficient addition effect is not achieved, and then the P/O ratio becomes small. On the other hand in case more cyclic ether is used than in the stated range, yield of the dihydric phenol decreases, and this is not desirable.

The reaction temperature may be in the range from 50° to 150°C, and preferably, in the range from 60° to 120°C. In case the temperature is lower than the stated range, the reaction rate becomes too slow; on the other hand in case the temperature is higher than the stated range, decomposition of the hydrogen peroxide or by-production of a high boiling material increases, the yield of dihydric phenol decreases. These results are undesirable.

Although the cyclic ether can be used as a solvent in the reaction of the present invention, it is preferable that a solvent other than the cyclic ether also be used in combination. As the other solvent, a polar solvent, such as methanol, ethanol, acetonitrile, acetone and water, may be used. Among these, water is especially preferable. The amount of the co-solvent used is not critical, but, when used in excess, the concentration of the reactant decreases, and then the reaction rate decreases; it is preferable, therefore, that the amount of the co-solvent used is within 1 time by weight of the phenols. Further, it is possible to control the P/O ratio within a certain range by varying the ratio of the cyclic ether and phenol which is used.

The method of the present invention can be carried out as either a batch or continuous process.

The present invention provides method for the preparation of dihydric phenols which is characterized in that the phenols are reacted with a hydrogen peroxide using a crystalline titanosilicate catalyst in the presence of a cyclic ether, and provides especially high para-selectivity compared with conventional methods, while at the same time, providing a high yield of dihydric phenols _versus_ hydrogen peroxide. Therefore, the present method is believed to have industrially important significance.

## EXAMPLES

Hereinafter, the method of the present invention is described in more concrete terms. The following examples, however, do not limit the method of the present invention. Further, the degree of conversion of hydrogen peroxide, and the yield of dihydric phenols which appear in the following examples and comparative examples are defined by the following formulas:

the degree of conversion of hydrogen peroxide (%) = [(number of moles of a hydrogen peroxide supplied - number of moles of a hydrogen peroxide unreacted) ÷

(number of moles of a hydrogen peroxide supplied)] × 100

the yield of dihydric phenols (%) = [(number of moles of dihydric

phenols produced) ÷ (number of moles of hydrogen peroxide converted)] × 100

## Preparation of the Catalyst

## TS-1 Catalyst

Into a flask having a internal volume of 200 ml and having four inlets, 45.5 g of a tetraethyl orthosilicate and 1.5 g of a tetraethyl orthotitanate were added under nitrogen flow, and dissolved homogeneously with stirring in an ice bath. After 100 g of 20% solution of a tetrapropylammonium hydroxide was added dropwise, carefully using a dropping funnel, the mixture rose gradually in temperature to room temperature, with stirring for 1 hour. The resulting colorless and homogeneous solution was heated to 80°C in an oil bath, and stirred for 5 hours, while removing ethanol formed by hydrolysis. After the termination of the hydrolysis, 60 ml of ion-exchanged water was added into the reaction mixture; thus the volume of the mixture was brought up to 150 ml. Next, the reaction mixture was charged into a 300 ml stainless steel autoclave, hydrothermally synthesized at 175°C, under its own pressure for 10 days. The thus formed white crystal were removed by centrifugation from the mother liquor, washed sufficiently with ion-exchanged water, then dried at 90°C for 6 hours. After drying sufficiently, the crystals were heat-treated in air at 550°C for 6 hours, crystalline titanosilicate was obtained (hereinafter refereed as TS-1). The X-ray diffraction and infrared spectrum of the recovered crystals were the same as are described in Japanese Patent Publication Kokoku No. 1-42889.

TS-2 Catalyst

Into a flask having an internal volume of 200 ml, and having four inlets, 45 g of a tetraethyl orthosilicate was charged under nitrogen flow, and a mixture of 64 g of a solution of 1 mole/l of tetrabutyl ammonium hydroxide in methanol with and 30 g of ion-exchanged water was added dropwise with sufficient stirring in an ice bath. After the addition, dry 2-propanol (10 g) solution of a tetrabutyl orthotitanate (2.23 g) was added dropwise with sufficient stirring in an ice bath.

To the thus obtained solution was added 50 g of ion-exchanged water, and the solution was heated to 80 °C in an oil bath, stirred for 3 hours, while removing ethanol, butanol, which were formed by hydrolysis, and the added 2-propanol. 50 g of ion-exchanged water was added into the reaction mixture; then the total volume of the mixture was brought up to 125 ml. Next, the reaction mixture was charged into a 300 ml stainless steel autoclave, hydrothermally synthesized at 170°C, under its own pressure for 10 days. After the hydrothermal synthesis, the same procedure as was used to obtain the TS-1 catalyst was used to obtain the crystalline titanosilicate, hereinafter referred to as TS-2. The X-ray diffraction and infrared spectrum of the recovered crystals were the same as the spectrum reported in J.D. Reddy.

Example 1

A stirrer, a cooler and a temperature indicator were provided to a flask having an internal volume of 200 ml and having four inlets; to this flask, 13.0 g of phenol, 4.0 g of 1,4-dioxane, 6.0 g of water and 1.3 g of TS-1 catalyst, prepared according to the above described procedure, were added, and heated to 70°C by an oil bath. After the temperature of the mixture reached 70°C, 3.0 g of 31 weight % of hydrogen peroxide was added dropwise using a quantitative pump over 2 hours, and stirred further for 20 minutes after the addition. The reaction solution was analyzed by liquid chromatography, and the yields of hydroquinone and catechol were calculated. The results are shown in Table 1.

Example 2

The same procedure was used as in Example 1, except that 2.0 g of 1,4-dioxane, and 8.0 g of water were used instead of 4.0 g of 1,4-dioxane, and 6.0 g of water as in Example 1. The results are shown in Table 1.

Comparative Example 1

The same procedure was used as in Example 1, except that 4.6 g of 1,4-dioxane was not added in Example 1. The results are shown in Table 1.

Examples 3 - 9

The same procedure was used as in Example 1, except that 1,4-dioxane, water, and TS-1 catalyst were used respectively in the amounts as described in Table 1. The results are shown in Table 1.

Examples 10 - 13

The same procedure was used as in Example 1, except that 0.6 g of TS-1 catalyst was used; further the amount of water added and the kind of cyclic ether used were changed as described in Table 1. The results are shown in Table 1.

Examples 14 - 16

The same procedure was used as in Example 1, except that 0.6 g of TS-1 catalyst, 5.0 g of 1,4-dioxane and 5.0 g of solvent were used as described in Table 1, instead of the 1.3 g of TS-1 catalyst, 4.0 g of 1,4-dioxane, and 6.0 g of water as were used in Example 1. The results are shown in Table 1.

Example 17

The same procedure was used as in Example 1, except that 0.6 g of the TS-1 catalyst, prepared according to the above procedure was used instead of the 1.3 g of TS-1 catalyst as was used in Example 1. The results are shown in Table 1.

Example 18

The same procedure was used as in Example 1, except that 14.8 g of anisole was used instead of the 13 g of phenol as was used in Example 1; also 0.6 g of TS-1 catalyst, 5.0 g of 1,4-dioxane, and 5.0 g of water were used. The results are shown in Table 1.

Table 1

| | Catalyst Added (g) | Cyclic Ether | Solvent | Amount Added (g) | | Degree of Conversion of Hydrogen Peroxide (%) | DHB Yield (%) | P/O Molar Ratio |
|---|---|---|---|---|---|---|---|---|
| | | | | Cyclic Ether | Solvent | | | |
| Ex. 1 | 1.3 | 1,4-dioxane | water | 4 | 6 | 100 | 75 | 7.3 |
| Ex. 2 | 1.3 | 1,4-dioxane | water | 2 | 8 | 100 | 79 | 4.0 |
| Ex. 3 | 0.6 | 1,4-dioxane | – | 10 | 0 | 100 | 43 | 4.4 |
| Ex. 4 | 0.6 | 1,4-dioxane | – | 5 | 0 | 100 | 57 | 5.3 |
| Ex. 5 | 0.6 | 1,4-dioxane | – | 2 | 0 | 100 | 46 | 1.9 |
| Ex. 6 | 0.6 | 1,4-dioxane | water | 5 | 5 | 100 | 71 | 6.9 |
| Ex. 7 | 0.6 | 1,4-dioxane | water | 2 | 8 | 100 | 75 | 4.4 |
| Ex. 8 | 0.6 | 1,4-dioxane | water | 0.5 | 9.5 | 100 | 67 | 2.0 |
| Ex. 9 | 5.2 | 1,4-dioxane | water | 5 | 5 | 100 | 70 | 5.4 |
| Ex. 10 | 0.6 | tetrahydropyran | water | 5 | 5 | 73 | 33 | 2.0 |
| Ex. 11 | 0.6 | tetrahydrofuran | – | 10 | 0 | 100 | 6 | 5.0 |
| Ex. 12 | 0.6 | tetrahydrofuran | water | 2 | 8 | 100 | 25 | 3.2 |
| Ex. 13 | 0.6 | 1,3-dioxane | water | 5 | 5 | 100 | 63 | 1.9 |
| Ex. 14 | 0.6 | 1,4-dioxane | acetone | 5 | 5 | 100 | 43 | 2.5 |
| Ex. 15 | 0.6 | 1,4-dioxane | methanol | 5 | 5 | 100 | 45 | 6.5 |
| Ex. 16 | 0.6 | 1,4-dioxane | acetonitrile | 5 | 5 | 100 | 33 | 2.7 |
| Ex. 17 | 0.6 | 1,4-dioxane | water | 5 | 5 | 100 | 45 | 4.6 |
| Ex. 18 | 0.6 | 1,4-dioxane | water | 5 | 5 | 82 | 9 | 11.0 |
| Com. Ex. 1 | 1.3 | none | water | 0 | 6 | 100 | 77 | 1.2 |

Note: DHB=diphenols

EP 0 548 807 B1

**Claims**

1.  A method for preparing dihydric phenols comprising reacting phenol or anisole with hydrogen peroxide to produce said dihydric phenols wherein said reaction is carried out in the co-presence of a crystalline titanosilicate catalyst and a cyclic ether.

2.  The method according to Claim 1, wherein said titanosilicate is pentasyl type titanosilicate.

3.  The method according to Claim 1, wherein said cyclic ether is selected from the group consisting of 1,3-dioxolane, tetrahydrofuran, tetrahydropyran, 1,3-dioxane, 1,4-dioxane and 1,3,5-trioxane.

4.  The method according to Claim 1, wherein said cyclic ether is 1,4-dioxane.

5.  The method according to Claim 1, wherein said cyclic ether is used in an amount of from 0.04 to 1.2 moles per 1 mole of phenol or anisole.

6.  The method according to Claim 1, wherein said cyclic ether is used in an amount of from 0.1 to 0.8 moles per 1 mole of phenol or anisole.

7.  The method according to Claim 1, which is characterized in that the reaction is carried out in a polar solvent which contains a cyclic ether.

8.  The method according to Claim 4, which is characterized in that the para- to ortho-isomer P/O ratio (P/O) is controlled by varying the ratio of the cyclic ether to the phenols or anisole.

9.  The method according to Claim 7, wherein said polar solvent is selected from the group consisting of water, acetone, methanol, acetonitrile and ethanol.

10. The method according to Claim 7, wherein said polar solvent is water.

11. The method according to Claim 1, wherein said hydrogen peroxide is used in an amount of not more than 0.5 per 1 mole of the phenol or anisole.

12. The method according to Claim 1, wherein said hydrogen peroxide is used in an amount of not more than 0.3 moles per 1 mole of the phenol or anisole.

13. The method according to Claim 1, wherein the reaction temperature is in the range of from 50° to 150°C.

14. The method according to Claim 1, wherein said titanosilicate catalyst is used in an amount in the range of from 0.02 to 0.8 g per 1 g of the phenol or anisole.

15. The method according to Claim 1, wherein said titanosilicate catalyst is used in an amount in the range of from 0.04 to 0.5 g per 1 g of the phenol of anisole.

**Patentansprüche**

1.  Ein Verfahren zur Herstellung von zweiwertigen Phenolen, welches die Reaktion von Phenol oder Anisol mit Wasserstoffperoxid umfaßt, um besagte zweiwertige Phenole herzustellen, wobei besagte Reaktion in der gleichzeitigen Gegenwart eines kristallinen Titanosilicat-Katalysators und eines zyklischen Ethers durchgeführt wird.

2.  Das Verfahren nach Anspruch 1, wobei besagtes Titanosilicat Titanosilicat vom Pentasyl-Typ ist.

3.  Das Verfahren nach Anspruch 1, wobei besagter zyklischer Ether ausgewählt ist aus der Gruppe, die aus 1,3-Dioxolan, Tetrahydrofuran, Tetrahydropyran, 1,3-Dioxan, 1,4-Dioxan und 1,3,5-Trioxan besteht.

4.  Das Verfahren nach Anspruch 1, wobei besagter zyklischer Ether 1,4-Dioxan ist.

5.  Das Verfahren nach Anspruch 1, wobei besagter zyklischer Ether in einer Menge von 0,04 bis 1,2 Mol pro 1 Mol Phenol oder Anisol verwendet wird.

8

**6.** Das Verfahren nach Anspruch 1, wobei besagter zyklischer Ether in einer Menge von 0,1 bis 0,8 Mol pro 1 Mol Phenol oder Anisol verwendet wird.

**7.** Das Verfahren nach Anspruch 1, das dadurch gekennzeichnet ist, daß die Reaktion in einem polaren Lösungsmittel durchgeführt wird, das einen zyklischen Ether enthält.

**8.** Das Verfahren nach Anspruch 4, das dadurch gekennzeichnet ist, daß das P/O-Verhältnis von para- zu ortho-Isomer (P/O) durch Variation des Verhältnisses des zyklischen Ethers zu den Phenolen oder dem Anisol gesteuert wird.

**9.** Das Verfahren nach Anspruch 7, wobei besagtes polares Lösungsmittel ausgewählt ist aus der Gruppe, die aus Wasser, Aceton, Methanol, Acetonitril und Ethanol besteht.

**10.** Das Verfahren nach Anspruch 7, wobei besagtes polares Lösungsmittel Wasser ist.

**11.** Das Verfahren nach Anspruch 1, wobei besagtes Wasserstoffperoxid in einer Menge von nicht mehr als 0,5 pro 1 Mol des Phenols oder Anisols verwendet wird.

**12.** Das Verfahren nach Anspruch 1, wobei besagtes Wasserstoffperoxid in einer Menge von nicht mehr als 0,3 Mol pro 1 Mol des Phenols oder Anisols verwendet wird.

**13.** Das Verfahren nach Anspruch 1, wobei die Reaktionstemperatur im Bereich von 50° bis 150°C liegt.

**14.** Das Verfahren nach Anspruch 1, wobei besagter Titanosilicat-Katalysator in einer Menge im Bereich von 0,02 bis 0,8 g pro 1 g des Phenols oder Anisols verwendet wird.

**15.** Das Verfahren nach Anspruch 1, wobei besagter Titanosilicat-Katalysator in einer Menge im Bereich von 0,04 bis 0,5 g pro 1 g des Phenols oder Anisols verwendet wird.

## Revendications

**1.** Procédé pour la préparation de phénols dihydriques comprenant la réaction de phénol ou d'anisol avec du peroxyde d'hydrogène pour produire lesdits phénols dihydriques, dans lequel ladite réaction est effectuée en présence simultanée d'un catalyseur titanosilicate cristallin et d'un éther cyclique.

**2.** Procédé selon la revendication 1, dans lequel ledit titanosilicate est un titanosilicate de type pentasyle.

**3.** Procédé selon la revendication 1, dans lequel l'éther cyclique est choisi dans le groupe constitué par le 1,3-dioxolane, le tétrahydrofurane, le tétrahydropyrane, le 1,3-dioxane, le 1,4-dioxane et le 1,3,5-trioxane.

**4.** Procédé selon la revendication 1, dans lequel l'éther cyclique est le 1,4-dioxane.

**5.** Procédé selon la revendication 1, dans lequel la quantité dudit éther cyclique utilisé est de 0,04 à 1,2 moles par mole de phénol ou d'anisole.

**6.** Procédé selon la revendication 1, dans lequel la quantité dudit éther cyclique utilisé est de 0,1 à 0,8 mole pour 1 mole de phénol ou d'anisole.

**7.** Procédé selon la revendication 1, caractérisé en ce que la réaction est effectuée dans un solvant polaire qui contient un éther cyclique.

**8.** Procédé selon la revendication 4, caractérisé en ce que le rapport (P/O) entre l'isomère para et l'isomère ortho P/O est contrôlé en faisant varier le rapport entre l'éther cyclique et les phénols ou l'anisole.

**9.** Procédé selon la revendication 7, dans lequel ledit solvant polaire est choisi dans le groupe constitué par l'eau, l'acétone, le méthanol, l'acétonitrile et l'éthanol.

**10.** Procédé selon la revendication 7, dans lequel le solvant polaire est l'eau.

**11.** Procédé selon la revendication 1, dans lequel la quantité de peroxyde d'hydrogène utilisé est au plus de 0,5 pour 1 mole de phénol ou d'anisole.

**12.** Procédé selon la revendication 1, dans lequel la quantité de peroxyde d'hydrogène utilisé est au plus de 0,3 mole pour 1 mole de phénol ou d'anisole.

**13.** Procédé selon la revendication 1, dans lequel la température de réaction est située dans l'intervalle entre 50° et 150°C.

**14.** Procédé selon la revendication 1, dans lequel la quantité de catalyseur titanosilicate utilisé est située dans l'intervalle entre 0,02 et 0,8 g pour 1 g de phénol ou d'anisole.

**15.** Procédé selon la revendication 1, dans lequel la quantité de catalyseur titanosilicate utilisé est située dans l'intervalle entre 0,04 et 0,5 g pour 1 g de phénol ou d'anisole.